# EUROPEAN PATENT SPECIFICATION

(11) **EP 3 081 238 B1**
(45) Date of publication and mention of the grant of the patent: **07.11.2018**
(21) Application number: 16160753.6
(22) Date of filing: 16.03.2016
(51) Int. Cl.: A61M 1/00

(54) **SUCTION-IRRIGATION HEAD**
SAUG- UND BEWÄSSERUNGSKOPF
TÊTE D'ASPIRATION/IRRIGATION

(30) Priority: 17.04.2015 TW 104112312; 17.04.2015 TW 104112313
(43) Date of publication of application: 19.10.2016
(73) Proprietor: ShineIN Biotechnology Co., Ltd., New Taipei City 242 (TW)
(72) Inventor: DAI, Lien-Guo, Xinzhuang District, New Taipei City 242 (TW); CHANG, Ya-Hui, Xinzhuang District, New Taipei City 242 (TW); DAI, Shiuan-Yuan, Xinzhuang District, New Taipei City 242 (TW); DAI, Shiuan-Jen, Xinzhuang District, New Taipei City 242 (TW); DAI, Shiuan-De, Xinzhuang District, New Taipei City 242 (TW)
(74) Representative: 2K Patentanwälte Blasberg Kewitz & Reichel

(56) References cited:
- EP-A1- 0 559 977
- WO-A1-00/29045
- WO-A1-2012/151751
- US-A- 2 568 566
- US-A- 5 779 662
- US-A1- 2005 171 468
- US-A1- 2009 204 065
- US-A1- 2013 261 600

## Description

### Technical Field

The present invention relates to a suction-irrigation head and, in particular, to a suction-irrigation head having two chambers.

### Background

During a surgical operation process, suction and irrigation are employed for cleansing a wound of a patient. To carry out suction and irrigation, multiple irrigation pipes are connected with their one end to an irrigation device, and the other end is inserted into the patient's wound. Then, multiple suction pipes are connected with their one end to a suction device, and the other end is inserted into the patient's wound. The irrigation device directs an irrigation liquid to wash out unwanted body fluid and tissues, and then the unwanted body fluid and tissues are drawn away out of the wound by means of multiple suction pipes.

However, in order to contain the multiple irrigation pipes and the multiple suction pipes, cutting has to be carried out on a patient to create a number of cut openings or a cut opening having a predetermined area or a larger area. The number and the area of the cut openings reflect the potential infection risk and the complexity of the operation procedures. Therefore, a key to improving a suction-irrigation method is to decrease the number and the area of the cut openings in order to reduce the surgical infection risk and the operation time. In addition, in order to ensure ideal functionality of a suction and irrigation system, a closed cavity is required. However, the junction of the conventional suction pipe and the cut opening often leaks fluid, so the suction and irrigation performance is not ideal, and taking care of the patients is troublesome.

Moreover, a conventional suction-irrigation head does not have a fixing mechanism, so it tends to slide, and thus the irrigation liquid fails to sufficiently cleanse a treatment portion.

Accordingly, the inventor made a lot of studies to improve the above-mentioned conventional technique and solve the problems, on the basis of which the present invention is accomplished.

WO 00/29045 A1 relates to an improved surgical instrument for the removal of fragmented tissues and fluids from a patient through a small incision. The instrument utilizes a source of suction, a source of irrigating fluid which suspends, dilutes the fragmented tissues that also provides a pressure vent to improve the speed, and thoroughness of the procedure. A multi-lumen cannula is used to deliver the irrigating fluid to the operative site, to vent the operative site, to remove the fragmented tissue, and fluids from the operative site.

US 2 568 566 A relates to therapeutic appliances are used in treating diseased body lesions or cavities. It is employed to withdraw hemorrhages, suppurations, fetid air or gas out of body lesions or cavities.

US 2005/171468 A1 relates to a medical intubation device for delivering tube feeds into the duodenum or other intestinal area while aspirating the stomach of a patient is inserted through the patient's oral-nasal cavity. A first lumen of a tubular assembly allows delivery of fluids through outlets into the intestines. A second lumen of the tubular assembly interior to the first lumen provides aspiration through outlets into the stomach. A third lumen of the tubular assembly interior to the second lumen provides ventilation of the second lumen. A weighted member can be coupled at the insertion end of the tubular assembly. A radio-opaque allows positioning of the outlets into the stomach.

WO 2012/151751 A1 relates to suction-irrigation system having a bi-lumen tip respectively coupled to a suction unit and a fluid delivery unit for removing unwanted materials from a body cavity and delivering fluids thereto is provided. The bi-lumen tip has a perforated end for inserting the bi-lumen tip into the body cavity and a non-perforated end for coupling the bi-lumen tip to the suction unit and the fluid delivery unit. The bi-lumen tip includes a drainage lumen and a fluid delivery lumen; the two lumens are arranged in parallel and share a common lumen wall within the bi-lumen tip. The drainage lumen is coupled to the suction unit, and has a first plurality of perforations on its surface for the removal of unwanted materials from the body cavity. The fluid delivery lumen is coupled to the fluid delivery unit via optionally connecting through an adapter, and has a second plurality of perforations on its surface for delivering the at least one fluid to the body cavity. The respectively first and second perforations of the drainage lumen and the fluid delivery lumen are disposed on part of the surface of the perforated end of the bi-lumen tip.

US 20130261600A1 relates to a surgical instrument including a main unit and a suction conduit. The main unit includes at least one tubular body having a distal end, a proximal end, and an inlet disposed near the distal end. The suction conduit is slidably disposed over the main unit, and is slidably movable along the main unit between a retracted position, at which the suction conduit does not cover the inlet of the main unit, and an extended position at which the suction conduit covers the inlet of the main unit and a suction inlet of the suction conduit is placed in fluid-communication with the main unit inlet. The surgical instrument can be used as a suction tool by placing the suction conduit in the extended position and applying a vacuum through the inlet of the main unit and the suction inlet.

### SUMMARY

It is an object of the present invention to provide a suction-irrigation head to provide irrigation and suction functions at the same time, whereby only one insertion position is required when using the suction-irrigation head, thereby reducing the surgical infection risk and saving the operation time.

Accordingly, the present invention provides a suction-irrigation head, comprising: a manifold set, the manifold set including a joint end at one end, the manifold set extending at the other end to form a discharge pipe and an intake pipe, an end of the discharge pipe forming a discharge opening, an end of the intake pipe forming an intake opening; a tube, the tube integrally extending from the joint end, the tube including a circumferential wall, a plurality of intake bores being disposed on one side of the circumferential wall, a plurality of discharge bores being disposed on the other side of the circumferential wall; and a separation wall, one end of the separation wall extending between the discharge pipe and the intake pipe, the other end of the separation wall extending from a distal end of the tube, the interior of the manifold set and the tube being divided by the separation wall to form an intake chamber and a discharge chamber not communicating with each other, the intake chamber, the intake pipe and the intake bores communicating with one another, the discharge chamber, the discharge pipe and the discharge bores communicating with one another.

The present invention also has the following features:
First, the intake chamber is smaller than the discharge chamber, so the irrigation liquid jets from the intake bores with a greater force, and unwanted body fluid and tissues can be accommodated in a bigger space when flowing to the discharge chamber, thereby improving the convenience of using the suction-irrigation head.
Second, the diameter sizes of the intake bores decrease in a direction from the distal end of the tube toward the manifold set, the diameter sizes of the discharge bores decrease in a direction from the distal end of the tube toward the manifold set, and thus the tube have good structural strength in use, thereby a lifespan of the suction-irrigation head is prolonged.
Third, the intake bores and the discharge bores are radially arranged on the circumferential wall to increase an irrigation area of the intake bores and a suction area of the discharge bores, thereby improving the convenience of using the suction-irrigation head. The present invention provides a two-chamber suction-irrigation head.

It is another object of the present invention to provide a two-chamber suction-irrigation head with a fixing mechanism to prevent fluid leakage.

The present invention further provides a suction-irrigation head comprising a tube. Two ends of the tube are a tube head portion and a tube end portion respectively, a separation wall longitudinally extends in the tube, the tube is divided by the separation wall to form an intake chamber and a discharge chamber separated from each other, an intake bore communicating with the intake chamber and an discharge bore communicating the discharge chamber are formed on a side surface of the tube head portion, an annular rib surrounds the side surface of the tube head portion, and the tube end portion branches and extends to form an intake pipe communicating with the intake chamber and a discharge pipe communicating with the discharge chamber.

It is preferable that there are multiple intake bores arranged longitudinally along the tube. The intake bores vary in diameter size, and the one of any two intake bores closer to a distal end of the tube head portion is larger in diameter size. The intake bores vary in opening direction, and the opening direction of the intake bores is arranged radially.

It is preferable that multiple discharge bores are disposed and arranged longitudinally along the tube. The discharge bores vary in diameter size, and the one of any two discharge bores closer to the distal end of the tube head portion is larger in diameter size. The discharge bores vary in opening directions, and the opening direction of the discharge bores is arranged radially.

It is preferable that the intake pipe forms an included angle with a longitudinal direction of the tube. The discharge pipe is arranged longitudinally along the tube. The intake bores and the discharge bores are respectively arranged at two opposite sides of the tube head portion, and the intake bores and the discharge bores are arranged in a staggered manner. The intake bores and the discharge bores are closer to the distal end of the tube head portion than the annular rib. An internal diameter of the intake chamber is smaller than an internal diameter of discharge chamber.

By means of the annular rib, the suction-irrigation head is fixed in a proper position to facilitate cleansing the treatment portion.

### BRIEF DESCRIPTION OF THE DRAWINGS

The disclosure will become more fully understood from the detailed description and the drawings given herein below for illustration only, and thus does not limit the disclosure, wherein:
FIG. 1 is a perspective view of a suction-irrigation head according to a first embodiment of the present invention;
FIG. 2 is a cross-sectional view of the suction-irrigation head according to the first embodiment of the present invention;
FIG. 3 is a schematic view illustrating the suction-irrigation head in use according to the first embodiment of the present invention;
FIG. 4 is a schematic view illustrating the suction-irrigation head in use according a second embodiment of the present invention;
FIG. 5 is a schematic view illustrating the suction-irrigation head in use according to a third embodiment of the present invention;
FIG. 6 is a perspective view of the suction-irrigation head according to a fourth embodiment of the present invention;
FIG. 7 is a cross-sectional view of the suction-irrigation head according to the fourth embodiment of the present invention;
FIG. 8 is a cross-sectional view of a tube head portion of the suction-irrigation head according to the fourth embodiment of the present invention; and
FIG. 9 is a schematic view illustrating the suction-irrigation head according to a fifth embodiment of the present invention.

### DETAILED DESCRIPTION

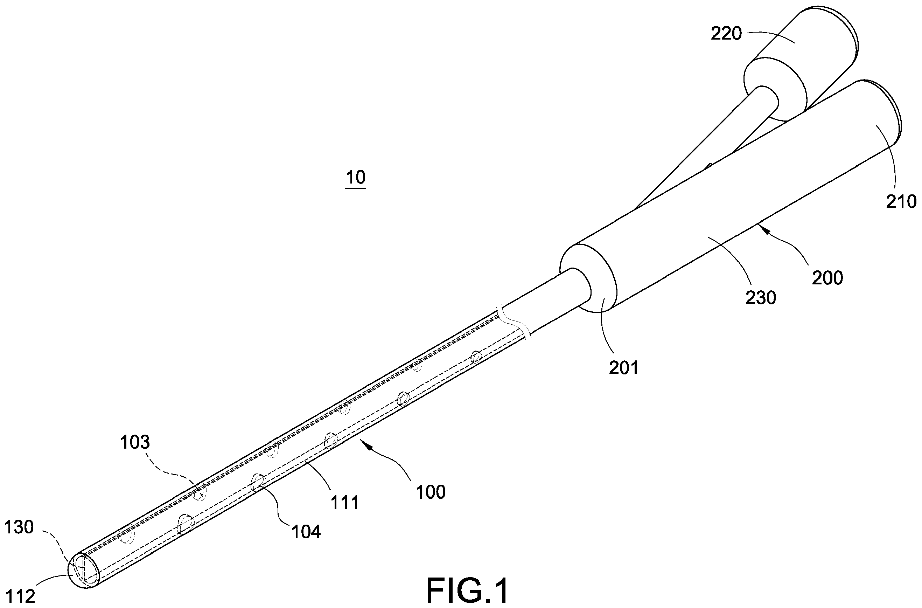

Referring to Figs. 1 to 3, a first embodiment of the present invention provides a suction-irrigation head. The suction-irrigation head comprises a tube 100, a manifold set 200, and a separation wall 130.

The manifold set 200 includes a joint end 201 at one end, and extends at the other end to form a discharge pipe 210 and an intake pipe 220. An end of the discharge pipe 210 forms a discharge opening 211, an end of the intake pipe 220 forms an intake opening 221. The manifold set 200 includes a tubular wall 230, the joint end 201 and the discharge pipe 210 are respectively formed at two ends of the tubular wall 230, and the intake pipe 220 integrally extends from the tubular wall 230. The manifold set 200 is a Y-shaped pipe or a T-shaped pipe; however, the present invention is not limited to this embodiment.

The tube 100 integrally extends from the joint end 201, the tube 100 includes a circumferential wall 111, a distal end of the tube 100 forms a closed end 112, a plurality of intake bores 103 are disposed on one side of the circumferential wall 111, a plurality of discharge bores 104 are disposed on the other side of the circumferential wall 111.

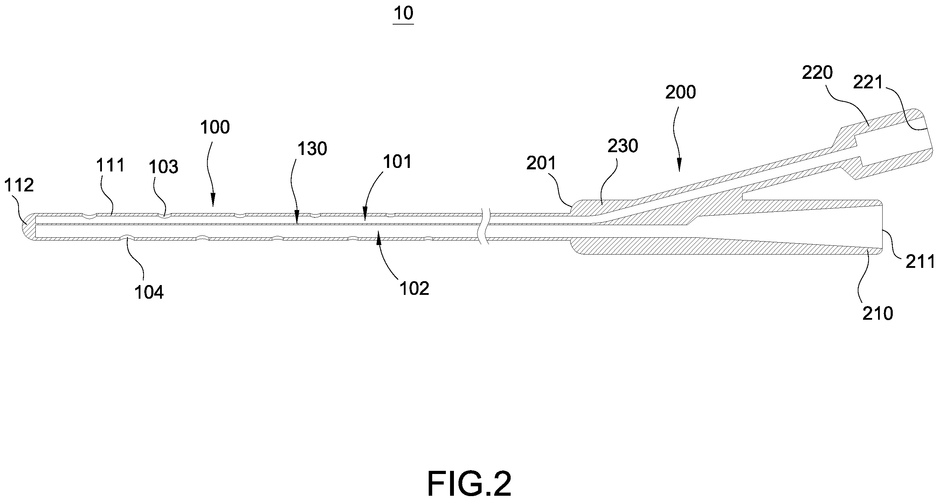

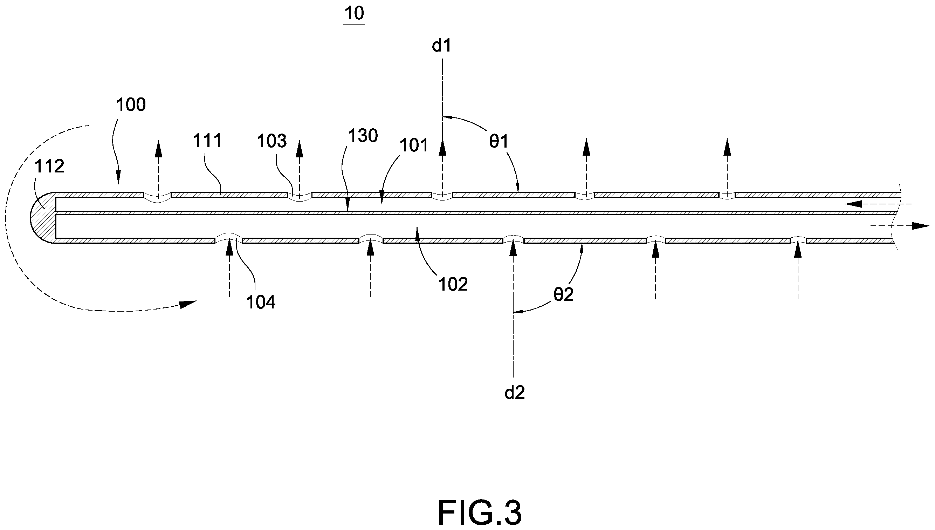

Referring to Figs. 2 and 3, the diameter sizes of the intake bores 103 decrease in a direction from the distal closed end 112 of the tube 100 toward the manifold set 200. The diameter sizes of the discharge bores 104 decrease in a direction from the distal closed end 112 of the tube 100 toward the manifold set 200.

Referring to Fig. 3, an opening direction d1 of each of the intake bores 103 forms a right angle θ1 with respect to the circumferential wall 111. An opening direction d2 of each of the discharge bores 104 forms a right angle θ2 with respect to the circumferential wall 111. The diameter size of each of the intake bores 103 and the discharge bores 104 ranges from 0.05 to 0.5 centimeters.

One end of the separation wall 130 extends between the discharge pipe 210 and the intake pipe 220, the other end of the separation wall 130 extends from the distal closed end 112 of the tube 100, the interior of the manifold set 200 and the tube 100 is divided by the separation wall 130 to form an intake chamber 101 and a discharge chamber 102 not communicating with each other, the intake chamber 101, the intake pipe 220 and the intake bores 103 communicate with one another, and the discharge chamber 102, the discharge pipe 210 and the discharge bores 104 communicate with one another. The intake chamber 101 is smaller than the discharge chamber 102.

Referring to Figs. 1 and 2, in the configuration of the suction-irrigation head 10 of the present invention, one end of the manifold set 200 defines the joint end 201, the other end of the manifold set 200 extends to form the discharge pipe 210 and the intake pipe 220, an end of the discharge pipe 210 forms the discharge opening 211, an end of the intake pipe 220 forms the intake opening 221. Furthermore, the tube 100 integrally extends from the joint end 201, the tube 100 includes a circumferential wall 111, the plurality of intake bores 103 are disposed on one side of the circumferential wall 111, and the plurality of discharge bores 104 are disposed on the other side of the circumferential wall 111. Moreover, one end of the separation wall 130 extends between the discharge pipe 210 and the intake pipe 220, the other end of the separation wall 130 extends from a distal end of the tube 100, the interior of the manifold set 200 and the tube 100 is divided by the separation wall 130 to form the intake chamber 101 and the discharge chamber 102 not communicating with each other, the intake chamber 101, the intake pipe 220 and the intake bores 103 communicate with one another, and the discharge chamber 102, the discharge pipe 210 and the discharge bores 104 communicate with one another.

Referring to Figs. 2 and 3, to use the suction-irrigation head 10 of the present invention, first the tube 100 is placed inside a wound of a patient, the discharge pipe 210 is connected to a suction device, the intake pipe 220 is connected to an irrigation device, the irrigation device directs an irrigation liquid to sequentially flow through the intake pipe 220, the intake chamber 101, and the multiple intake bores 103 to wash out unwanted body fluid and tissues. Finally, the suction device draws away the unwanted body fluid and tissues out of the wound through, sequentially, the multiple discharge bores 104, the discharge chamber 102, and the intake pipe 220.

Since the interior of the tube 100 is divided by the separation wall 130 to form the intake chamber 101 and the discharge chamber 102 not communicating with each other, the suction-irrigation head 10 provides an irrigation function and a suction function at the same time. Therefore, when using the suction-irrigation head 10, only one cut opening is required for insertion of the suction-irrigation head 10, thereby having advantages of reducing the surgical infection risk and reducing the surgical operation time.

Furthermore, the intake chamber 101 is smaller than the discharge chamber 102, so that the irrigation liquid jets from the intake bores 103 with a greater force, and the unwanted body fluid and tissues can be accommodated in a bigger space when flowing to the discharge chamber 102, thereby improving convenience of using the suction-irrigation head 10.

Moreover, when using the suction-irrigation head 10, bending occurs frequently at positions where the tube 100 is adjacent to the manifold set 200. If the diameter size of the intake bore 103 or the discharge bore at the bend is too large, it causes stress concentration, deformation and damage. Therefore, the diameter sizes of the multiple intake bores 103 decrease in a direction from the distal closed end 112 of the tube 100 toward the manifold set 200. The diameter sizes of the multiple discharge bores also decrease in a direction from the distal closed end 112 to the tube 100 toward the manifold set 200. Therefore, the tube 100 has better structural strength in use, and a lifespan of the suction-irrigation head 10 is prolonged.

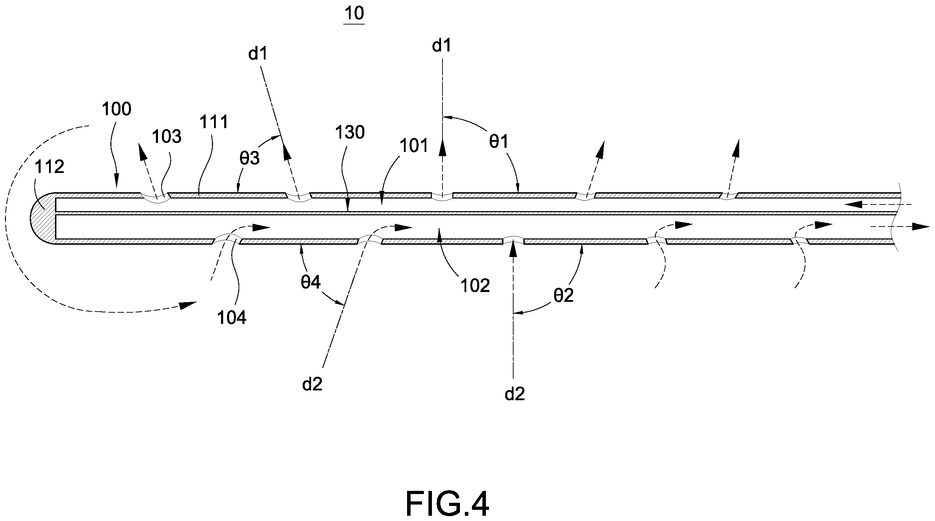

Referring to Fig. 4, it illustrates the suction-irrigation head 10 according to a second embodiment of the present invention. This embodiment of Fig. 4 is - similar to the above-mentioned embodiment of Figs. 1 to 3 with the difference that an opening direction d1 of each of the intake bores 103 forms a right angle θ1 or an acute angle θ3 with respect to the circumferential wall 111, and an opening direction d2 of each of the discharge bores 104 forms a right angle θ2 or an acute angle θ4 with respect to the circumferential wall 111.

In detail, the opening direction d1 of the middle intake bores 103 forms the right angle θ1 with respect to the circumferential wall 111, and the opening direction d1 of the intake bores 103 arranged at two sides forms the acute angle θ3 with respect to the circumferential wall 111. The opening direction d2 of the middle discharge bores 104 forms the right angle θ2 with respect to the circumferential wall 111, and the opening direction d2 of the discharge bores 104 arranged at two sides forms the acute angle θ4 with respect to the circumferential wall 111. Accordingly, the multiple intake bores 103 and the multiple discharge bores 104 are radially arranged on the circumferential wall 111 to increase an irrigation area of the intake bores 103 and a suction area of the discharge bores 104, thereby improving the convenience of using the suction-irrigation head 10.

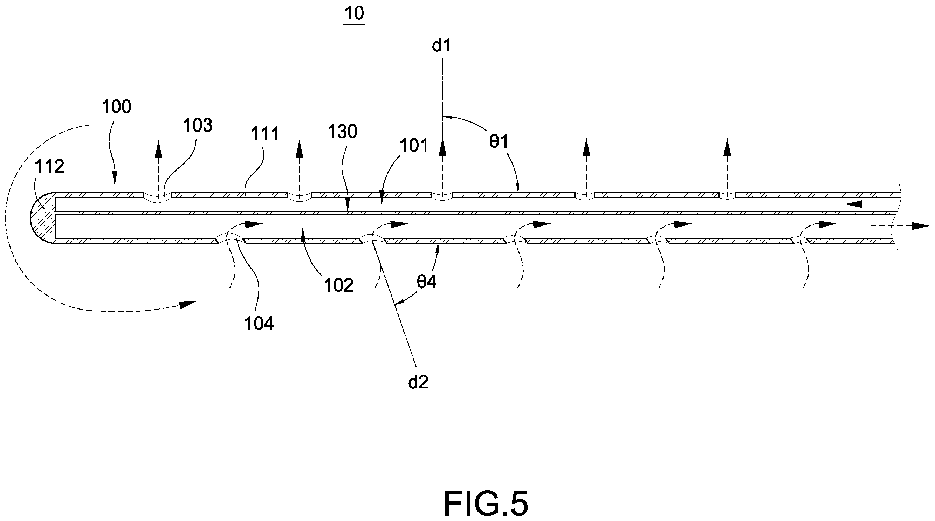

Referring to Fig. 5 which illustrates the suction-irrigation head 10 according to a third embodiment of the present invention, the embodiment of Fig. 5 is similar to the embodiment of Figs. 1 to 3 with the difference that the opening direction d2 of each of the discharge bores 104 forms the acute angle θ4 with respect to the circumferential wall 111.

In detail, the opening direction d2 of each of the discharge bores 104 forms the acute angle θ4 with respect to the circumferential wall 111, so the flowing of the unwanted body fluid and tissues to the discharge chamber 102 encounters greater resistance, thereby the speed of the unwanted body fluid and tissues flowing to the discharge chamber 102 is decreased to allow the irrigation liquid jetting from the intake holes 103 to have more time for irrigation, thus preventing the irrigation liquid from being quickly drawn away out of the wound.

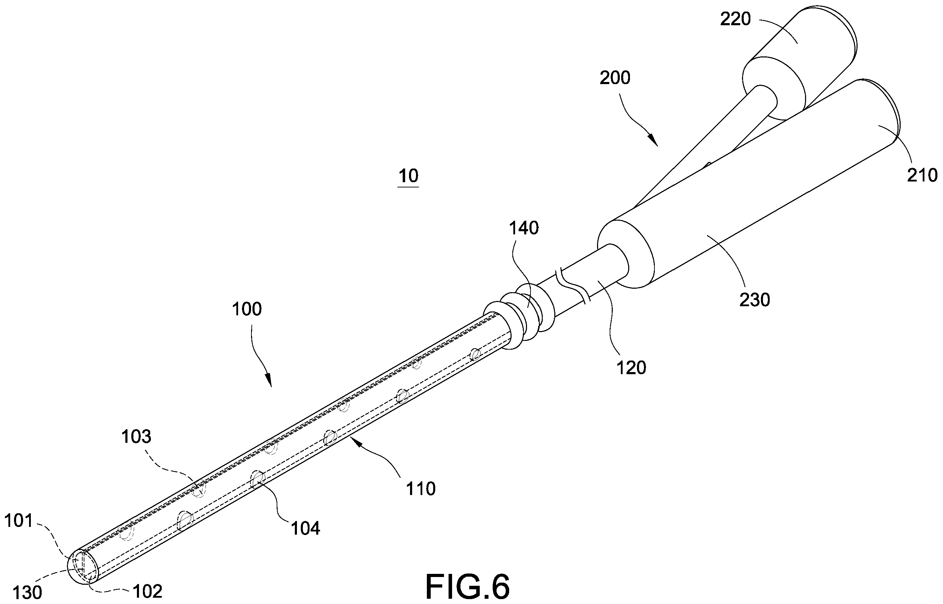

Referring to Figs. 6 to 8, a fourth embodiment of the present invention provides a suction-irrigation head 10 for placed inside the body of a patient to cleanse a wound/opening inside the same. According to the present embodiment, the suction-irrigation head 10 comprises a tube 100. Preferably, the tube 100 is made of silicone and bendable; however, the present invention is not limited to any particular material. Two ends of the tube 100 are respectively a tube head portion 110 and a tube end portion 120. A separation wall 130 is disposed in the tube 100. The separation wall 130 extends longitudinally along the tube 100, so the tube 100 is divided by the separation wall 130 to form an intake chamber 101 and a discharge chamber 102 separated from each other. The intake chamber 101 and the discharge chamber 102 are of elongated shape and pass throughout the tube 100, An internal diameter of the discharge chamber 101 is preferably smaller than the internal diameter of the discharge chamber 102.

Multiple intake bores 103 and multiple discharge bores 104 are formed on a surface of the tube head portion 110 of the tube 100, the intake bores 103 and the discharge bores 104 are respectively arranged at opposite sides of the tube head portion 110. The intake bores 103 and the discharge bores 104 are arranged longitudinally along the tube 100, and the intake bores 103 and the discharge bores 104 are arranged in a staggered manner.

Each of the intake bores 103 communicates the intake chamber 101, the intake bores 103 vary in diameter size, and the one of any two intake bores 103 closer to a distal end of the tube head portion 110 is larger in diameter size. The discharge bores 104 vary in diameter size, and the one of any two discharge bores 104 closer to the distal end of the tube head portion 110 is larger in diameter size.

At least one annular rib 140 surrounds a side surface of the tube head portion 110 of the tube 100, and, preferably, multiple annular ribs 140 are disposed on the side surface of the tube head portion 110; however, the present invention is not limited to any particular number of the annular ribs 140. It is preferable that the intake bores 103 and the discharge bores 104 are closer to the distal end of the tube head portion 110 than the annular rib 140.

An intake pipe 200 and a discharge pipe 210 branch and extend from the tube end portion 120 of the tube 100, and the intake pipe 200 communicates with the intake chamber 101, and the discharge pipe 210 communicates with the discharge chamber 102. In the present embodiment, the discharge pipe 210 is arranged longitudinally along the tube 100, and the intake pipe 200 forms an included angle with a longitudinal direction of the tube 100.

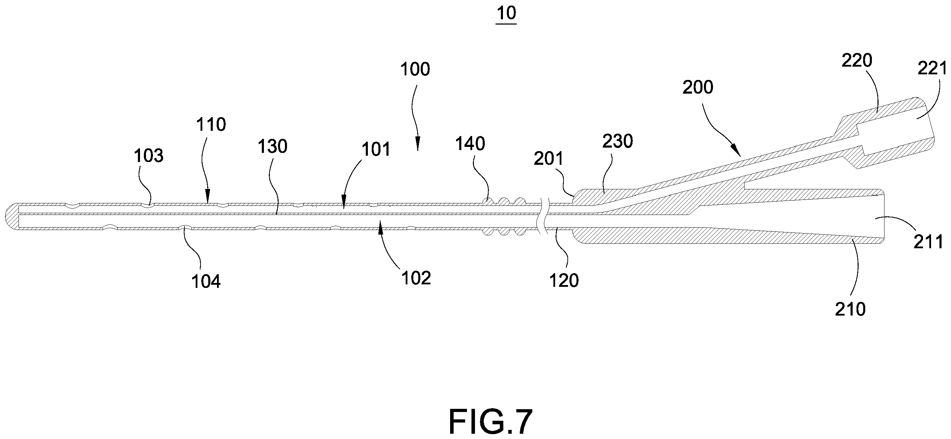

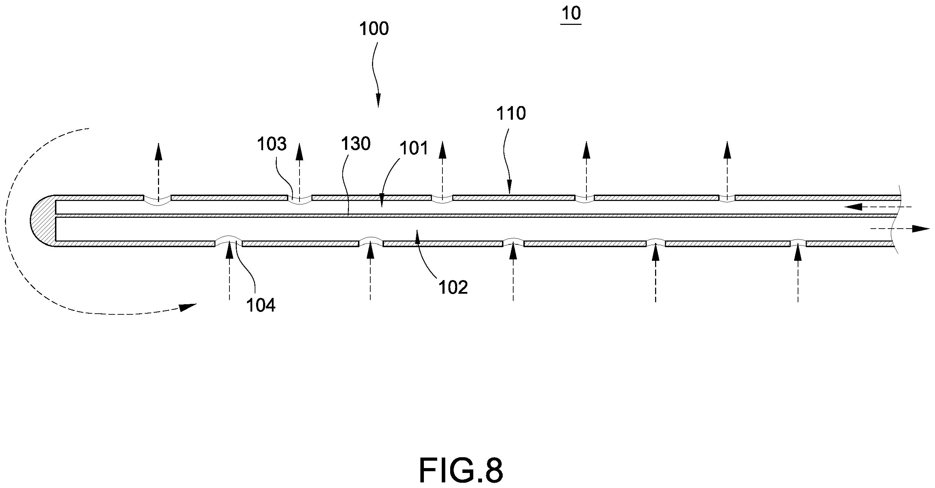

Referring to Figs. 7 and 8, when using the suction-irrigation head of the present invention, the tube head portion 110 of the tube 100 is inserted into the body of a patient to reach a treatment portion, the tube head portion 110 is engaged, preferably by means of the annular rib 140, inside the body of the patient and fixed to the treatment portion. The tube end portion 120 of the tube 100 is disposed outside of the patient's body for medical staff's operation to cleanse the treatment portion. The medical staff feed an irrigation liquid into the intake pipe 200, so the irrigation liquid passes through the intake pipe 200 to flow through the intake chamber 101 to the tube head portion 110, and the irrigation liquid passes through each intake bore 103 to flow out of the tube head portion 110 to reach and cleanse the treatment portion. The used irrigation liquid passes through each discharge bore 104 to flow into the discharge chamber 102 to be discharged out of the treatment portion. It is preferable that the annular rib 140 can obstruct the irrigation liquid from flowing out via the wound. It is also preferable that the discharge pipe 210 can be connected to a negative pressure source to enable flowing of the irrigation liquid; however, the present invention is not limited in this regard.

The suction-irrigation head 10 of the present invention utilizes the annular rib 140 to fix the tube head portion 110 in a position, so as to cleanse the treatment portion. Moreover, when the tube 100 is inserted into the patient's body, the stress applied to the tube head portion 110 increases from the distal end thereof. The intake bores 103 and the discharge bores 104 vary in diameter size from large to small, and the diameter size decreases from the distal end of the tube head portion 110, so that a stress limit of the intake bores 103 (or the discharge bores 104) increases from the distal end of the tube head portion 110, thus preventing the intake bores 103 or the discharge bores 104 from being damaged by bending the tube head portion 110. Moreover, the internal diameter of the intake chamber 101 is preferably smaller than the internal diameter of the discharge chamber 102, so the irrigation liquid flows faster in the intake chamber 101, whereby the irrigation liquid can effectively cleanse the treatment portion when it flows to the treatment portion, and discharging of the irrigation liquid via the discharge chamber 102 is also facilitated. The discharge pipe 210 is arranged longitudinally along the tube 100, the intake pipe 200 forms an included angle with a longitudinal direction of the tube 100, so the flow resistance in the discharge pipe 210 is smaller than the flow resistance in the intake pipe 200, thereby facilitating discharging the irrigation liquid via the discharge chamber 102.

A fifth embodiment of the present invention provides a suction-irrigation head 10 for insertion into a patient's body to cleanse a wound inside the body. A structure of the suction-irrigation head 10 is similar to the structure of the above-mentioned fourth embodiment, so similarities are not repeated for brevity. The difference between the present embodiment and the fourth embodiment is detailed as follows.

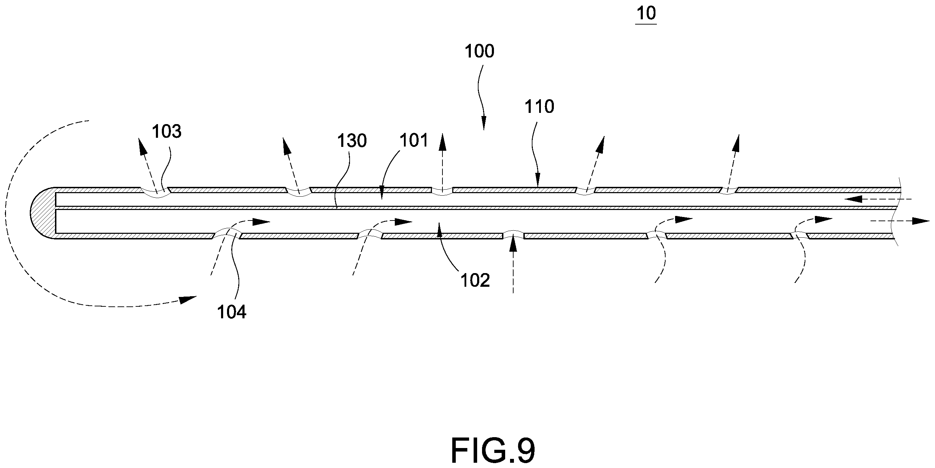

Referring to Fig. 9, the intake bores 103 vary in opening direction, and the opening direction of the intake bores 103 is arranged radially. The discharge bores 104 vary in opening direction, and the opening direction of the discharge bores 104 is arranged radially. This configuration facilitates effectively flowing and diffusion of the irrigation liquid when the irrigation liquid flows to the treatment portion, thereby the irrigation liquid can efficiently cleanse everywhere of the treatment portion.

## Claims

1. A suction-irrigation head (10), comprising a tube (100), two ends of the tube (100) being a tube head portion (110) and a tube end portion (120) respectively, a separation wall (130) longitudinally extending along the tube (100), the tube (100) being divided by the separation wall (130) to form an intake chamber (101) and a discharge chamber (102) separated from each other, a plurality of intake bores (103) communicating with the intake chamber (101) and a plurality of discharge bores (104) communicating the discharge chamber (103) being formed on a side surface of the tube head portion (110), the tube end portion (120) branching and extending to form an intake pipe (220) communicating with the intake chamber (101) and a discharge pipe (210) communicating with the discharge chamber (102),
**characterized in that** an annular rib (140) surrounding the side surface of the tube head portion (110),
wherein the diameter size of the intake bores (103) and the discharge bores (104) decrease in a direction from the distal end of the tube (100) toward the manifold set (200), thereby preventing deformation and damage to the tube (100) due to concentration of stress.

2. The suction-irrigation head of claim 1, wherein the intake bores (103) vary in opening direction, and the opening direction of the intake bores (103) is arranged radially.

3. The suction-irrigation head of any of the claims 1 to 2, wherein the discharge bores (104) vary in opening direction, and the opening direction of the discharge bores (104) is arranged radially.

4. The suction-irrigation head of any of the claims 1 to 3, wherein the intake bores (103) and the discharge bores (104) are closer to a distal end of the tube head portion (110) than the annular rib (140).

5. The suction-irrigation head of any of the claims 1 to 4, wherein an internal diameter of the intake chamber (101) is smaller than an internal diameter of discharge chamber (102).

## Patentansprüche

1. Saug-Spülkopf (10), umfassend ein Rohr (100), wobei zwei Enden des Rohres (100) jeweils ein Rohrkopfabschnitt (110) und ein Rohrendabschnitt (120) sind, eine Trennwand (130), die sich in Längsrichtung entlang des Rohres (100) erstreckt, wobei das Rohr (100) durch die Trennwand (130) geteilt ist, um eine Einlasskammer (101) und eine voneinander getrennte Auslasskammer (102) zu bilden, eine Vielzahl von Einlassbohrungen (103), die mit der Einlasskammer (101) in Verbindung stehen, und eine Vielzahl von Auslassbohrungen (104) die mit der Auslasskammer (103) in Verbindung stehen, die auf einer Seitenfläche des Röhrenkopfabschnitts (110) ausgebildet sind, wobei der Rohrendabschnitt (120) verzweigt und sich erstreckt, um ein Einlassrohr (220), das mit der Einlasskammer (101) in Verbindung steht, und ein Auslassrohr (210), das mit der Auslasskammer (102) in Verbindung steht, zu bilden,
**dadurch gekennzeichnet**, durch eine ringförmige Rippe (140), die die Seitenfläche des Rohrkopfabschnittes (110) umgibt,
wobei die Durchmessergröße der Einlassbohrungen (103) und der Auslassbohrungen (104) in einer Richtung vom distalen Ende des Rohres (100) in Richtung des Verteilersatzes (200) abnehmen, wodurch Verformungen und Beschädigungen des Rohres (100) aufgrund von Spannungskonzentrationen vermieden werden.

2. Saug-Spülkopf nach Anspruch 1, wobei die Einlassbohrungen (103) in öffnungsrichtung variieren und die Öffnungsrichtung der Einlassbohrungen (103) radial angeordnet ist.

3. Saug-Spülkopf nach einem der Ansprüche 1 bis 2, wobei die Auslassbohrungen (104) in Öffnungsrichtung variieren und die öffnungsrichtung der Auslassbohrungen (104) radial angeordnet ist.

4. Saug-Spülkopf nach einem der Ansprüche 1 bis 3, worin die Einlassbohrungen (103) und die Auslassbohrungen (104) näher an einem distalen Ende des Rohrkopfabschnitts (110) als die ringförmige Rippe (140) liegen.

5. Saug-Spülkopf nach einem der Ansprüche 1 bis 4, wobei ein Innendurchmesser der Einlasskammer (101) kleiner ist als ein Innendurchmesser der Auslasskammer (102).

## Revendications

1. Une tête d'aspiration-irrigation (10), comprenant un tube (100), deux extrémités du tube (100) étant une partie de la tête de tube (110) et une partie terminale de tube (120) respectivement, une paroi de séparation (130) s'étendant longitudinalement le long du tube (100), le tube (100) étant divisé par la paroi de séparation (130) pour constituer une chambre d'admission (101) et une chambre d'évacuation (102) séparées l'une de l'autre,
une pluralité d'alésages d'entrée (103) communiquant avec la chambre d'admission (101) et une pluralité d'alésages d'évacuation (104) communiquant avec la chambre d'évacuation (103) formés sur une surface latérale de la partie de tête de tube (110), la partie terminale de tube (120) se branchant et s'étendant pour former un tuyau d'admission (220) communiquant avec la chambre d'admission (101) et un tuyau d'évacuation (210) communiquant ave la chambre d'évacuation (102),
**caractérisée en ce qu'**une nervure annulaire (140) entoure la surface latérale de la partie de tête de tube (110),
dans laquelle le diamètre des alésages d'entrée (103) et des alésages d'évacuation (104) décroît suivant une direction partant de l'extrémité distale du tube (100) vers l'ensemble collecteur (200), empêchant ainsi une déformation et un endommagement du tube (100) lors de contraintes élevées.

2. La tête d'aspiration-irrigation (10) de la revendication 1, dans laquelle les alésages d'entrée (103) varient dans leur direction d'ouverture, et la direction d'ouverture des alésages d'entrée (130) est radiale.

3. La tête d'aspiration-irrigation (10) de l'une quelconque des revendications 1 ou 2, dans laquelle les alésages d'évacuation (104) varient dans leur direction d'ouverture, et la direction d'ouverture des alésages de d'évacuation (104) est disposée radiale.

4. La tête d'aspiration-irrigation (10) de l'une quelconque des revendications 1 à 3, dans laquelle les alésages d'entrée (103) et les alésages d'évacuation (104) sont plus proches d'une extrémité distale de la partie de tête de tube (1100) que la nervure annulaire (140).

5. La tête d'aspiration-irrigation (10) de l'une quelconque des revendications 1 à 4, dans laquelle un diamètre interne de la chambre d'admission (101) est plus petit qu'un diamètre interne de la chambre d'évacuation (102).
